# EUROPEAN PATENT APPLICATION

(11) **EP 1 798 649 A1**
(43) Date of publication of application: **20.06.2007**
(21) Application number: 05111286.0
(22) Date of filing: 25.11.2005
(51) Int. Cl.: G06F 19/00

(54) **Medical image display and review system**

(71) Applicant: Agfa HealthCare NV, 2640 Mortsel (BE)
(72) Inventor: Van der Linden, Mario, c/o AGFA-GEVAERT, 2640, Mortsel (BE); Vande Casteele, Alessi, c/o AGFA-GEVAERT, 2640, Mortsel (BE); Verstraeten, Luc c/o AGFA-GEVAERT, 2640, Mortsel (BE); Ferrant, Matthieu c/o AGFA-GEVAERT, 2640, Mortsel (BE); Friedrichkeit, Sepp c/o AGFA-GEVAERT, 2640, Mortsel (BE)
(74) Representative: Theunis, Patrick

(57) **Abstract**

An image display and review system for display of medical images represented by a digital image data set wherein a pre-defined number of viewports for display of different image representations is provided and wherein at least some of these viewports are configured to enable sequential display of different image representations deduced from the same digital image data set.

## Description

### FIELD OF THE INVENTION

The present invention relates to an image display and review system suitable for displaying and reviewing medical images such as lung images.

### BACKGROUND OF THE INVENTION

In state of the art display systems for display and review of medical images such as CT images, a standard axial CT view is commonly displayed on a display screen.

If a physician is interested in other types of views such as coronal view or an image with a different window level setting etc. , appropriate image representations are to be reconstructed on the CT scanner and retrieved via a picture archiving and communication system (PACS) connecting the CT scanner and the display and review station.

This mode of operation is inconvenient, time-consuming and cumbersome.

Furthermore in existing systems these different representations are displayed in a large number of juxtaposed viewports which might result in the need of more than one display screen to enable display of all required viewports. Furthermore, due to the large number of viewports to be displayed, the dimensions of an individual viewport will be small which makes the total overview less clear.

It is an object of the present invention to provide a display and review system which overcomes the disadvantages of the prior art systems.

### SUMMARY OF THE INVENTION

The above-mentioned objects are realized by a display and review system having the specific features set out in claim 1.

Specific features for preferred embodiments of the invention are set out in the dependent claims.

The image display and review system of the present invention is suitable for several kinds of medical applications such as display and review of lung images, e.g. to detect nodules and/or polyps in the lung, or display and review of thorax images etc.

The image display and review system according to the present invention provides that a full examination involving review of a large set of different displayed representations of the image information can be performed on a single screen.

By selecting a small number of different viewports (windows) for the display, typically four (4) viewports, with the possibility to change the displayed views within the viewports, the operator disposes of all required image representations to perform an examination on a single screen. Moreover the dimensions of the different images can be adequately large to provide clear images.

Examples of the displayed views in the viewports are shown in the enclosed screen prints and are explained below. Typically the user can jump from one image representation to another within one viewport by means of mouse clicks or other types of user interfacing means such as cursor control.

For example for a lung nodule detection application, four viewports can be shown.
The user can toggle in a first viewport from an axial MIP (maximum intensity projection) to a coronal or sagittal view.
Different window/level settings may be applied.

In another viewport similar image representations can be displayed so that the user can compare corresponding locations in different image representations displayed in juxtaposed viewports.
In a third view port a rotatable topogram of air filled regions with bronchi can for example be shown.
In a fourth view port a spherical rotatable GPU (Graphical processing unit) based volume rendered MIP (maximum intensity projection) of a suspected region with a suspect element in the center can for example be displayed.

Preferably the image display and review system according to the present invention is coupled to a picture archival and communication system (PACS) and is set up to load and process image data from the PACS into a display and review application running on said display and review system, the application being organized to deduce from the loaded data said different image representations by processing the image data.
In this way the application becomes modality independent and is applicable to all DICOM-compliant images.

In an alternative embodiment images from more than one data set can be displayed and reviewed.
In one embodiment the different data sets relate to follow up images originating from the same modality (linked data).
In another embodiment the different data sets relate to images originating from another modality.

Further advantages and embodiments of the present invention will become apparent from the following description and drawings.

Another aspect of the present invention relates to a display and review method for display and review of medical images comprising the steps of
- loading a digital data set representing a medical image from a picture archiving and communication system (PACS) into a display and review application,
- deducing from said digital data set a number of different image representations,
- displaying said deduced image representations in a number of viewports, at least some of these viewports being configured to enable sequential display of some of said different image representations.

Examples of different image representations which may be deduced from the digital data set have been disclosed higher with regard to the image display and review system.

Sequential display may be enabled through user intervention, e.g. by mouse click or cursor control.

Preferably the viewports are displayed on a single display screen.

The embodiments of the method of the present invention are generally implemented in the form of a computer program product adapted to carry out the method steps of the present invention when run on a computer.

The computer program product is commonly stored in a computer readable carrier medium. Alternatively the computer program product takes the form of an electric signal and can be communicated to a user through electronic communication.

### BRIEF DESCRIPTION OF THE DRAWINGS

Fig. 1 shows a screen print illustrating a standard view layout according to the present invention,
Fig. 2 shows a screen print illustrating another standard view layout according to the present invention.

### DETAILED DESCRIPTION OF THE INVENTION

The present invention is described with reference to a lung nodule display and reviewing application but is not limited to this specific application.

In the following explanations several abbreviations are used which are explained below:
MIP is an abbreviation of maximum intensity projection,
AveIP is an abbreviation of average intensity projection,
MinIP is an abbreviation of minimum intensity projection,
GPU is an abbreviation of graphical processing unit,
PACS is an abbreviation of picture archiving and communication system.

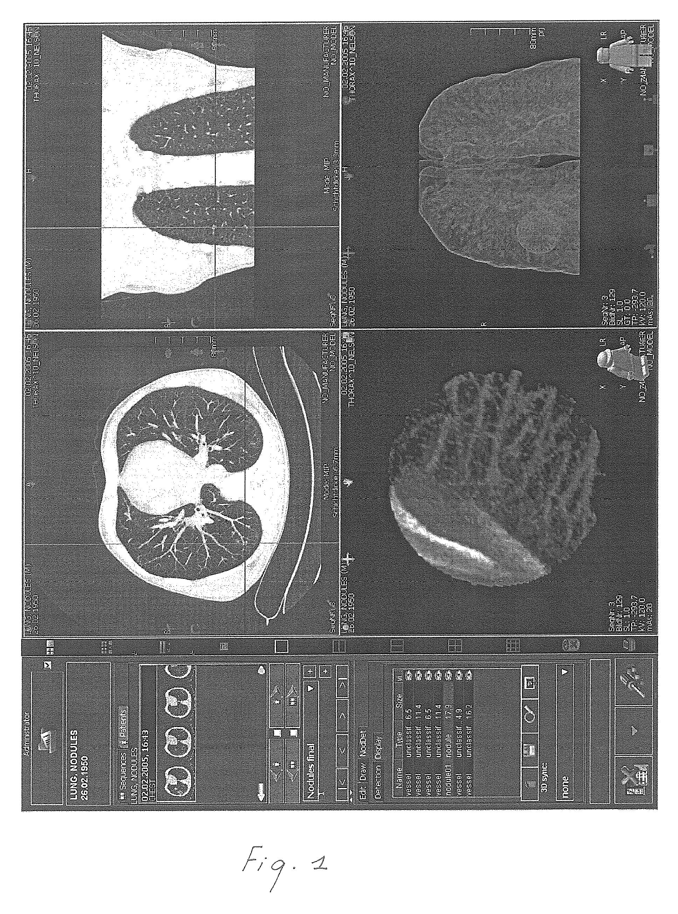

Figure 1 is a standard view lay-out of with suspect element list and window views set to (clockwise from top left) (a) MIP of 6.7 mm thickness, (b) coronal MIP of 3.3 mm, (c) topogram air filled view, (d) spherical rotatable GPU based volume rendered spherical MIP of suspect element with surrounding region in colour "off" mode.

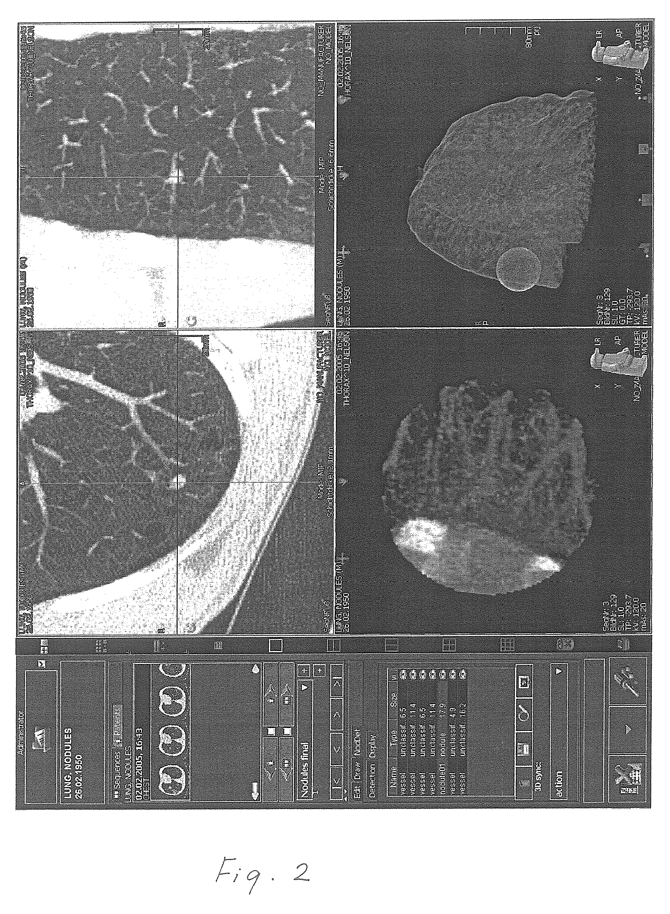

Figure 2 is a standard view layout with suspect element list left and window views set to (clockwise from top left) (a) MIP of 2.1 mm thickness with automatic zoom on suspect element, (b) coronal view with MIP of thickness 6.6 mm with automatic zoom on suspect element (c) topogram airfilled view, rotation angle linked to spherical MIP in bottom left view window, (d) spherical rotatable GPU based volume rendered spherical MIP of suspect element with surrounding region in colour "off" mode.

According to the present invention a user interface is provided which enables the user to toggle in a viewport from one representation of the image to another, for example from an axial MIP (maximum intensity projection) to a coronal or sagittal view. Suitable means enabling said user interaction are for example mouse clicks or cursor movements.

A toolbar can be provided which may contain:
a) a list of detected nodules characterized by name, classification, size and viewed status (seen/not seen)
b) a tab for choosing window-level presets of the top two) views: mediastinum, lung and bone setting
c) algorithm search criteria settings e.g. in semi-automatic mode, i.e. the user can choose size and density characteristics of the suspect areas to be found, prior to the running of the algorithm.

A specific embodiment of a workflow for a lung nodule display and reviewing application is described below.
1) In a first step a user loads image data from thorax CT images into a display and review software application running on a display and review system which is coupled to a picture archiving and communication system (PACS).
   The data are preferably DICOM compatible.
2) The next step may be the automatic segmentation of air-filled regions within the image of the lungs. An air-filled region mask is created by forming a lung border mask containing the entire lung region and subtracting a mediastinum region mask. To include the lung borders the final mask is dilated.
3) An automatic lung nodule enhanced viewing algorithm indicates spherical structures, e.g. of 4-20 mm, with densities ranging between set thresholds within the segmented areas.
4) Next a suspect element list may be created.
5) The volume size of suspect elements may be determined and displayed on the suspect element list.
6) Found suspect elements may be classified, described and named for future reporting. This can be e.g. be performed by double clicking on an element in the list and by choosing a fitting classification from a default list and/or typing in the description and name.
7) A spherical user rotatable GPU based volume-rendered MIP may be created for a suspected region preferably with the suspect element in the center.
8) The location of a nodule and the surrounding region of the MIP may be displayed on a topogram air filled view.
9) The user can create new suspect areas with automatic creation of spherical user-rotatable GPU based volume-rendered MIP.
10) The user can delete suspect elements from the list.
11) The user can also restore the listing of all suspect elements in the list found by the algorithm by pressing a reset button.
12) The user can save suspect areas, defined by the algorithm and/or defined by the user.
13) The user can jump from one suspect element to another by clicking on the suspect areas displayed on the topogram view.
14) The user can jump from one suspect element to another by clicking on the suspect element in the suspect elements list.
15) There is also the possibility for automatic zoom and centralized focus on suspect elements in top view (axial, sagittal/coronal) when the user jumps to an element in the list or on the topogram view.

## Claims

1. An image display and review system for display of images represented by a digital image data set comprising
a pre-defined number of viewports for display of different image representations,
at least some of said viewports being configured to enable sequential display of different image representations deduced from the same digital image data set.

2. An image display and review system according to claim 1 wherein said pre-defined number of viewports is displayed on a single display screen.

3. An image display and review system according to claim 1
- being coupled to a picture archival and communication system (PACS) and
- being organized to load and process image data from said PACS into a display and review application running on said display and review system, and
- said display and review application being organized to deduce from the loaded data said different image representations.

4. An image display and review system according to any one of the preceding claims wherein said image is a lung image.

5. A display and review method for display and review of medical images comprising the steps of
- loading a digital data set representing a medical image from a picture archiving and communication system (PACS) into a display and review application,
- deducing from said digital data set a number of different image representations,
- displaying said deduced image representations in a number of viewports, at least some of these viewports being configured to enable sequential display of some of said different image representations.

6. A display and review method according to claim 5 wherein said viewports are displayed on a single display screen.

7. A display and review method according to claim 5 wherein said image is a lung image.

8. A computer program product adapted to carry out the steps of any of claims 5 to 7 when run on a computer.

9. A computer readable carrier medium comprising computer executable program code adapted to carry out the steps of any of claims 5 to 7.
